# EUROPÄISCHE PATENTSCHRIFT

(11) **EP 2 755 581 B1**
(45) Veröffentlichungstag und Bekanntmachung des Hinweises auf die Patenterteilung: **05.12.2018**
(21) Anmeldenummer: 12756511.7
(22) Anmeldetag: 12.09.2012
(51) Int. Cl.: A61B 17/29, A61B 17/00

(54) **CHIRURGISCHES ROHRSCHAFTINSTRUMENT**
SURGICAL TUBULAR SHAFT INSTRUMENT
INSTRUMENT CHIRURGICAL À TIGE TUBULAIRE

(30) Priorität: 12.09.2011 DE 102011053517
(43) Veröffentlichungstag der Anmeldung: 23.07.2014
(73) Patentinhaber: Aesculap AG, 78532 Tuttlingen (DE)
(72) Erfinder: PLEIL, Elke, 78073 Bad Dürrheim (DE); KUPFERSCHMID, Bernhard, 78576 Emmingen-Liptingen (DE)
(74) Vertreter: Hoeger, Stellrecht & Partner Patentanwälte mbB
(86) Internationale Anmeldenummer: PCT/EP2012/067791
(87) Internationale Veröffentlichungsnummer: WO 2013/037801

(56) Entgegenhaltungen:
- EP-A1- 0 702 937
- EP-A2- 2 163 216
- WO-A1-97/13462
- WO-A2-2009/073577
- DE-A1- 10 132 358
- US-A- 5 755 723
- US-A1- 2004 243 125
- US-A1- 2007 287 993

## Beschreibung

Die vorliegende Erfindung betrifft ein chirurgisches Rohrschaftinstrument mit einem Griffteil, einem rohrförmigen Schaft mit einem proximalen und einem distalen Ende, mit einem am distalen Ende des Schafts angeordneten Werkzeug sowie mit einem Kraftübertragungselement, wobei das Kraftübertragungselement in dem Schaft geführt angeordnet ist und zum Übertragen einer Betätigungskraft vom Griffteil auf das Werkzeug zum Einen mit dem Griffteil, zum Anderen mit dem Werkzeug gekoppelt ist. Der Schaft ist lösbar mittels einer Kupplungsvorrichtung an dem Griffteil gehalten.

Chirurgische Rohrschaftinstrumente der eingangs beschriebenen Art kommen insbesondere bei endoskopischen chirurgischen Eingriffen zum Einsatz. Sind sie als wieder verwendbare Rohrschaftinstrumente ausgebildet, so lassen sich insbesondere der Schaft und das Kraftübertragungselement mit dem Werkzeug vom Griffteil und der Kupplungsvorrichtung trennen. Nach der Reinigung wird das Instrument sterilisiert und wieder montiert. Bei Rohrschaftinstrumenten, bei denen das Griffteil mit Einwegschäften bestückt wird, wird nach der Reinigung das Griffteil sterilisiert und mit einem neuen Einwegschaft bestückt. Bei den so genannten Single-Port-Access-Techniken wird in einer Öffnung im Körper des Patienten ein Trokar gesetzt, über welchen zwei oder mehr Instrumente in das Körperinnere des Patienten eingeführt werden.

Um - wie bei herkömmlichen endoskopischen Operationen üblich - eine Triangularität der Instrumente zu erhalten, sind die Schäfte der Instrumente nach bestimmten Vorgaben gebogen ausgebildet. Damit kann mit den zwei oder mehr Instrumenten an derselben Stelle im Operationsbereich gearbeitet werden. Typischerweise weisen die Instrumente dabei einen Schaft mit einem im Wesentlichen geraden Abschnitt auf, der im Trokar geführt ist, während ein distaler Abschnitt mit dem Werkzeug zunächst von dem geraden Abschnitt abweicht, um mit seinem distalen Ende gegebenenfalls wieder in Richtung zur verlängerten Achse des geraden Abschnitts zurückzukehren. Solche Instrumente sind beispielsweise aus der WO 2006/019723 A2 oder auch der WO 2009/073577 A2 bekannt.

Häufig ist bei solchen Rohrschaftinstrumenten der Schaft drehfest an der Kupplungsvorrichtung gehalten, während diese wiederum drehbar am Griffteil gelagert ist. Damit lassen sich für den Chirurgen optimale Arbeitsbedingungen schaffen. Eine hierfür geeignete Kupplungsvorrichtung ist beispielsweise aus der DE 199 35 042 A1 bekannt.
Rohrschaftinstrumente mit einer Arretiervorrichtung zum Festlegen der Drehposition des Schaftes gegenüber dem Griffteil sind beispielsweise aus US 2007/0287993 A1, US 5,755,723 A1, DE 101 32 358 A1 oder EP 0 702 937 A1 bekannt.

Die Aufgabe der vorliegenden Erfindung ist es, ein verbessertes chirurgisches Rohrschaftinstrument vorzuschlagen, mit dem einfacher und auch sicherer gearbeitet werden kann.

Diese Aufgabe wird durch ein chirurgisches Rohrschaftinstrument der vorliegenden Erfindung mit den Merkmalen des Anspruchs 1 gelöst.

Aufgrund der erfindungsgemäß zusätzlich vorhandenen Arretiervorrichtung lässt sich die Drehposition des Schafts gegenüber dem Griffteil festlegen, so dass auch die Lage oder Stellung des Werkzeugs gegenüber dem Griffteil einstellbar und fixierbar ist.

Soweit im Zusammenhang mit der Beschreibung der Erfindung von Drehbarkeit, einer Drehfestigkeit oder einer Drehposition gesprochen wird, wird - soweit im jeweiligen Zusammenhang nicht anders erläutert - auf die Längsachse des Schafts, insbesondere des proximalen, mit dem Griffteil verbundenen Abschnitts des Schafts Bezug genommen, die die Lage der Drehachse im Wesentlichen bestimmt.

Bei einem gebogenen Schaft oder einem gegenüber dem Schaft abgewinkelten Werkzeug ist damit eine definierte Position der Werkzeuge und ihrer Bewegungen bei Krafteinleitung durch das Griffteil gegenüber diesem festgelegt und ein sicheres Arbeiten möglich, insbesondere auch, wenn bei einem Arbeitsschritt größere Kräfte aufgewendet werden müssen.

Vorzugsweise ist die Arretiervorrichtung so gestaltet, dass die Drehposition des Schafts gegenüber dem Griffteil in vorgegebenen Winkelinkrementen festgelegt werden kann.

Bei einer weiter bevorzugten Ausführungsform der Erfindung ist die Arretiervorrichtung lösbar am Instrument befestigbar. Alternativ kann auch vorgesehen sein, dass die Arretiervorrichtung am Instrument fixiert ist und lediglich beim Auseinanderbauen des Instruments, insbesondere beim Trennen des Schafts vom Griffteil, abnehmbar ist.

Erfindungsgemäß weist das Instrument eine Kupplungsvorrichtung auf, die drehbar am Griffteil gehalten ist, wobei das proximale Ende des Schafts lösbar und drehfest an der Kupplungsvorrichtung gehalten ist. Die Drehposition der Kupplungsvorrichtung ist mittels der Arretiervorrichtung gegenüber dem Griffteil entsprechend der vorgegebenen Drehposition des Schafts gegenüber dem Griffteil, insbesondere in vorgegebenen Winkelinkrementen, festlegbar.

Die Arretiervorrichtung ist mit einem proximalen und einem distalen Ende und einem dazwischen angeordneten Mittelabschnitt ausgebildet, wobei das distale Ende am proximalen Ende des Schafts oder an der Kupplungsvorrichtung befestigbar ist, wobei das proximale Ende der Arretiervorrichtung mit dem Griffteil lösbar und drehfest verbindbar ist und wobei die Kupplungsvorrichtung und der Mittelabschnitt der Arretiervorrichtung miteinander in Eingriff bringbar sind, wodurch die Drehposition der Kupplungsvorrichtung gegenüber dem Griffteil fixiert wird.

Auch das distale Ende der Arretiervorrichtung lässt sich lösbar an der Kupplungsvorrichtung bzw. dem proximalen Ende des Schafts verbinden. Diese Verbindung kann drehbar oder drehfest ausgebildet sein. Vorzugsweise wird das distale Ende der Arretiervorrichtung im Klemmsitz am Schaft oder an der Kupplungsvorrichtung gehalten.

Ist das distale Ende der Arretiervorrichtung mit dem Schaft bzw. dem Kupplungsteil fest verbunden, dann wird diese Verbindung drehbar ausgestaltet.

Bei einer anderen Ausführungsform kann auch das proximale Ende der Arretiervorrichtung mit dem Instrument fest verbunden sein, d.h. in diesem Fall mit dem Griffteil.

Eine feste Verbindung zwischen Arretiervorrichtung und Instrument hat insbesondere den Vorteil, dass die Arretiervorrichtung im zusammengebauten Zustand des Rohrschaftinstruments unverlierbar an diesem gehalten wird.

Bevorzugt wird bei den Ausführungsformen, bei denen eines der proximalen und distalen Enden der Arretiervorrichtung mit dem Instrument verbunden ist, auch mindestens eines der Enden der Arretiervorrichtung schwenkbar mit dem Mittelabschnitt verbunden. Damit lässt sich das andere Ende der Arretiervorrichtung von dem Instrument lösen, die Kupplungsvorrichtung zusammen mit dem Schaft drehen und danach die Arretiervorrichtung mit ihrem anderen Ende wieder am Instrument festlegen und damit die neu eingestellte Drehposition der Kupplungsvorrichtung gegenüber dem Griffteil festlegen.

Vorzugsweise ist dann das jeweils andere Ende, beispielsweise das proximale Ende, der Arretiervorrichtung im Klemmsitz am Griffteil gehalten bzw. das distale Ende der Arretiervorrichtung im Klemmsitz an der Kupplungsvorrichtung bzw. am Schaft gehalten.

Hierzu wird das im Klemmsitz zu befestigende Ende der Arretiervorrichtung vorzugsweise gabelförmig ausgebildet und liegt im Falle des proximalen Endes der Arretiervorrichtung am Griffteil zur Herstellung einer drehfesten Verbindung an zwei gegenüber liegenden Oberflächen desselben an.

Auch das andere Ende der Arretiervorrichtung lässt sich gabelförmig ausbilden, wobei im Falle des distalen Endes die Kupplungsvorrichtung bzw. der Schaft in einem Umfangswinkel von mehr als 180° umgriffen wird. Hier ergibt sich eine sichere Verbindung auch dann, wenn die Arretiervorrichtung im zusammengesetzten Zustand des Instruments vollständig von diesem getrennt werden kann.

Diese Variante der Arretiervorrichtung eignet sich vor allem auch für die Umrüstung herkömmlicher Griffteile.

Vorzugsweise weist der Mittelabschnitt der Arretiervorrichtung ein Bügelteil auf, welches in eine Ausnehmung der Kupplungsvorrichtung einrückbar ist. Das Bügelteil und die Ausnehmung sind vorzugsweise im Wesentlichen komplementär ausgebildet, so dass eine drehfeste Verbindung resultiert. Um mehrere Drehpositionen in definierten Winkelinkrementen festlegen zu können, weist bei einer solchen Ausgestaltung des erfindungsgemäßen Rohrschaftinstruments die Kupplungsvorrichtung mehrere solcher Ausnehmungen auf. Vorzugsweise umfasst die Kupplungsvorrichtung mehrere über ihren Umfang gleichmäßig verteilt angeordnete Vorsprünge, wobei zwischen jeweils zwei benachbarten Vorsprüngen die Ausnehmungen ausgebildet sind.

Alternativ kann der Mittelabschnitt der Arretiervorrichtung eine Ausnehmung aufweisen, die optional als eine durchgehende Ausnehmung ausgestaltet ist, in welche ein Vorsprung der Kupplungsvorrichtung einrückbar ist. Die Kupplungsvorrichtung weist dabei vorzugsweise mehrere in Umfangsrichtung regelmäßig angeordnete Vorsprünge auf, insbesondere vier bis sieben Vorsprünge. Damit lässt sich wieder die Drehposition in vorgegebenen Winkelinkrementen gegenüber dem Griffteil festlegen.

Bei einer bevorzugten Ausführungsform des erfindungsgemäßen Rohrschaftinstruments ist die Arretiervorrichtung als Kunststoffteil, insbesondere als Spritzgussteil, ausgebildet.

Alternativ kann die Arretiervorrichtung des erfindungsgemäßen Rohrschaftinstruments auch als Blechteil ausgebildet sein.

Wie zuvor erwähnt, ist der Schaft des erfindungsgemäßen Rohrschaftinstruments vorzugsweise mit einem mittleren, im Wesentlichen geraden Abschnitt ausgebildet und weist einen gegenüber dem mittleren Abschnitt abgewinkelten distalen Abschnitt oder ein gegenüber dem distalen Abschnitt abgewinkeltes Werkzeug auf.

Ist der distale Abschnitt abgewinkelt, weist dieser vorzugsweise eine im Wesentlichen kreisbogenförmige Gestalt auf, wobei das distale Ende des Schafts in der Flucht des mittleren Abschnitts angeordnet ist.

Auch der proximale Abschnitt des Schafts kann gegenüber dem mittleren Abschnitt abgewinkelt ausgebildet sein. Insbesondere bevorzugt ist ein abgewinkelter proximaler Abschnitt mit einer im Wesentlichen s-förmigen Gestalt, wobei dann weiter vorzugsweise der proximale Endbereich des proximalen Abschnitts des Schafts in einem spitzen Winkel zum mittleren Abschnitt des Schafts ausgerichtet ist.

Es hat sich darüber hinaus als vorteilhaft gezeigt, wenn das Längenverhältnis des proximalen zum mittleren Abschnitt des Schafts ca. 1:2 bis ca. 1:3 beträgt. Des Weiteren ist vorteilhaft, wenn das Längenverhältnis vom mittleren zum distalen Abschnitt des Schafts ca. 2:1 bis ca. 3:1 beträgt.

Diese und weitere Vorteile der Erfindung werden im Folgenden anhand der Zeichnung noch näher erläutert. Es zeigen im Einzelnen:
- Figur 1A: eine erste Ausführungsform eines erfindungsgemäßen chirurgischen Rohrschaftinstruments;
- Figur 1B: eine zweite Ausführungsform eines erfindungsgemäßen chirurgischen Rohrschaftinstruments;
- Figuren 2A und 2B: ein Griffteil eines erfindungsgemäßen chirurgischen Rohrschaftinstruments mit einer ersten Ausführungsform einer Arretiervorrichtung;
- Figuren 3A und 3B: ein Griffteil eines erfindungsgemäßen chirurgischen Rohrschaftinstruments mit einer zweiten Ausführungsform einer Arretiervorrichtung;
- Figuren 4A und 4B: ein Griffteil eines erfindungsgemäßen chirurgischen Rohrschaftinstruments mit einer dritten bzw. vierten Ausführungsform einer Arretiervorrichtung;
- Figuren 5A bis 5C: ein Griffteil eines erfindungsgemäßen chirurgischen Rohrschaftinstruments mit einer fünften Ausführungsform einer Arretiervorrichtung; und
- Figuren 6A und 6B: ein Griffteil eines erfindungsgemäßen chirurgischen Rohrschaftinstruments mit einer sechsten Ausführungsform einer Arretiervorrichtung.

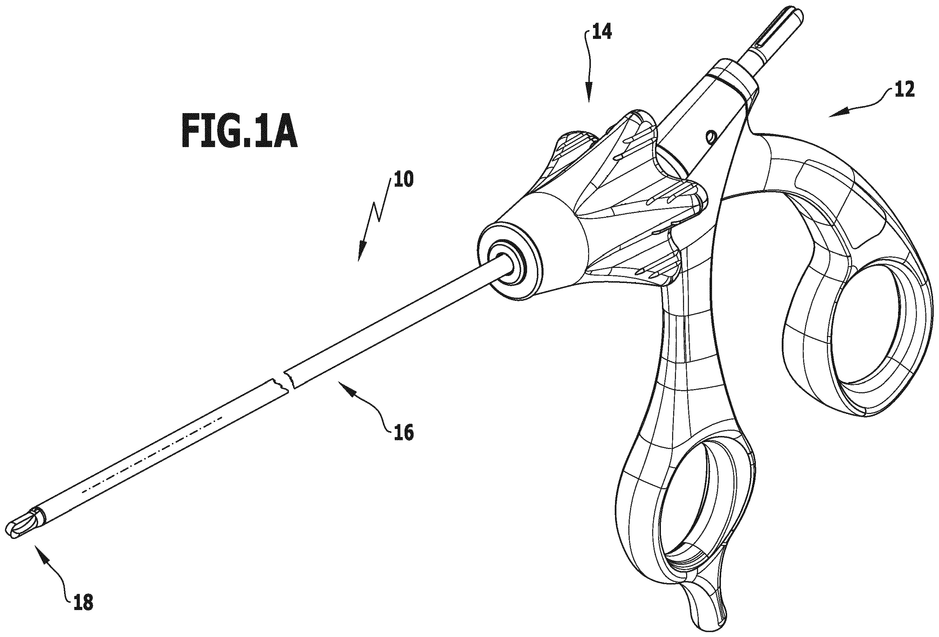

Die Figur 1A zeigt ein insgesamt mit dem Bezugszeichen 10 versehenes erfindungsgemäßes chirurgisches Rohrschaftinstrument mit einem Griffteil 12, einer Kupplungsvorrichtung 14 sowie einem Schaft 16, der an seinem distalen Ende ein Werkzeug 18 trägt.

Die Kupplungsvorrichtung ist, wie in Figur 1A gezeigt, als so genannter Drehstern ausgebildet, der mit fünf in gleichen Winkeln verteilten Vorsprüngen an seinem dem Griffteil 32 zugewandten Ende ausgestattet ist, während das dem Schaft 36 zugewandte distale Ende in der Regel kegelstumpfförmig ausgebildet ist.

Das Werkzeug 18 ist in einer geraden Anordnung gegenüber dem geraden Schaft 16 gezeigt, kann aber auch in einer abgewinkelten Position gegenüber dem Schaft 16 angeordnet sein, um mit dem Instrument einen größeren Aktionsradius darzustellen.

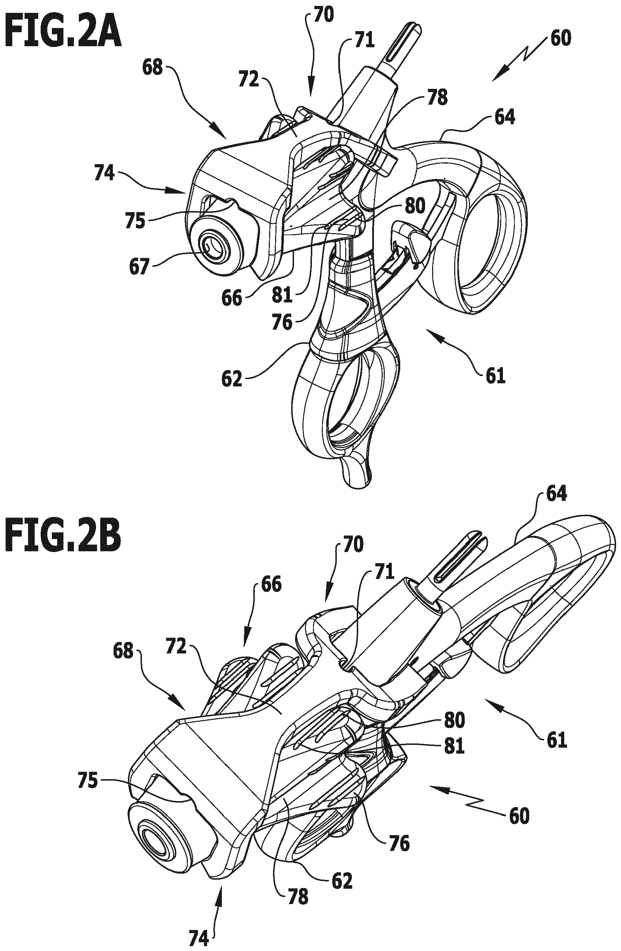

Der Schaft 16 mit dem Werkzeug 18 ist drehfest in der Kupplungsvorrichtung 14 gehalten, während die Kupplungsvorrichtung 14 wiederum drehbar am Griffteil 12 angeordnet ist. So lässt sich die Stellung des Werkzeugs gegenüber dem Griffteil in der für den chirurgischen Eingriff günstigsten Drehstellung einstellen. Zur Fixierung der gewählten Einstellung dient eine Arretiervorrichtung, die in Figur 1A der Einfachheit halber weggelassen ist und im Zusammenhang mit den Figuren 2 bis 6 in verschiedenen Ausführungsformen beschrieben wird.

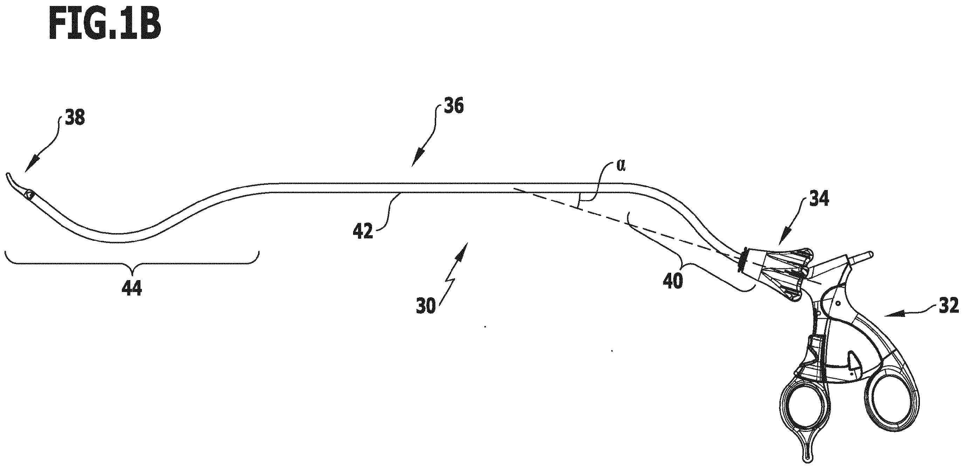

Figur 1B zeigt ein erfindungsgemäßes chirurgisches Rohrschaftinstrument 30 mit einem Griffteil 32, einer Kupplungsvorrichtung 34, einem Schaft 36 und einem Werkzeug 38. Der Einfachheit halber ist in der Darstellung der Figur 1B die Arretiervorrichtung weggelassen. Auch hier darf auf die Beschreibung der Figuren 2 bis 6 verwiesen werden.

Erneut ist der Schaft 36 drehfest an der Kupplungsvorrichtung 34 gehalten, während diese wiederum drehbar an dem Griff 32 gelagert ist.

Die Kupplungsvorrichtung 34 ist, wie auch in Figur 1A gezeigt, als so genannter Drehstern ausgebildet, der mit fünf in gleichen Winkeln verteilten Vorsprüngen an seinem dem Griffteil 32 zugewandten Ende ausgestattet ist, während das dem Schaft 36 zugewandte distale Ende in der Regel kegelstumpfförmig ausgebildet ist.

Anders als bei der Figur 1A gezeigt, ist der Schaft 36 in drei unterschiedliche Abschnitte, nämlich einen proximalen Abschnitt 40, einen mittleren, im Wesentlichen geraden Abschnitt 42 sowie einen distalen Abschnitt 44 untergliedert. Der proximale Abschnitt 40 ist im Wesentlichen s-förmig gebogen, derart, dass das proximale Ende des proximalen Abschnitts 40 in einem spitzen Winkel zur Längsachse des mittleren Abschnitts 42 ausgerichtet ist. In dem gezeigten Ausführungsbeispiel beträgt der Winkel α ca. 15°.

Der distale Abschnitt 44 weist eine Kreisbogenform auf, derart, dass das distale Ende des Schafts 36 ungefähr in der Verlängerung der Längsachse des mittleren Abschnitts 42 positioniert ist.

Während das chirurgische Instrument in den Figuren 1A und 1B wie erwähnt der Einfachheit halber ohne Arretiervorrichtung gezeigt ist, sei diese nun anhand der folgenden Figuren 2 bis 6 in mehreren Variationen anhand bevorzugter Ausführungsbeispiele erläutert.

Die Figuren 2A und 2B zeigen ein erfindungsgemäßes Instrument 60 mit einem Griffteil 61 mit einer feststehenden Griffbranche 62 und einer daran schwenkbar gelagerten Griffbranche 64 sowie eine an der Griffbranche 62 drehbar montierte Kupplungsvorrichtung 66. Die Kupplungsvorrichtung 66 weist an dem distalen Ende eine Öffnung 67 auf, in der ein Schaft des Rohrschaftinstruments (hier nicht dargestellt) drehfest aufgenommen werden kann.

Der Schaft ist zusammen mit der Kupplungsvorrichtung 66 gegenüber dem Griffteil 61 drehbar und lässt sich in vorgegebenen Winkelpositionen gegenüber dem Griffteil 61 mittels einer Arretiervorrichtung 68 fixieren.

Die Kupplungsvorrichtung 66 ist wieder als so genannter Drehstern ausgebildet und weist distalseitig eine Kegelstumpfform auf, während proximalseitig von der Umfangsfläche fünf sternförmig abstehende Vorsprünge 76 ausgebildet sind, zwischen denen Vertiefungen 78 vorhanden sind.

Bei dem gezeigten Ausführungsbeispiel des Griffteils 61 ist die Arretiervorrichtung 68 mit einem gabelförmigen proximalen Ende 70, einem bügelförmigen mittleren Abschnitt 72 sowie einem gabelförmigen distalen Ende 74 ausgestattet.

Der Mittelabschnitt 72 der Arretiervorrichtung 68 ist bügelförmig ausgebildet und weist eine Breite auf, die zur Breite der Vertiefung 78 korrespondiert, d.h. komplementär ausgebildet ist.

Zur genaueren Fixierung des Drehsterns 66 gegenüber dem Bügel 72 und auch zur Verbesserung der Griffigkeit des Drehsterns 66 weisen die Vorsprünge 76 auf beiden Flanken Rippen 80, 81 auf. Der mittlere Abschnitt 72 der Arretiervorrichtung 68 ist so bemessen, dass er zwischen die an den Flanken unten liegenden Rippen 81 zweier Vorsprünge 76 passt, so dass sich eine sehr genaue Positionierung und Fixierung der Winkelposition der Kupplungsvorrichtung 66 gegenüber dem Griffteil 61 im montierten Zustand der Arretiervorrichtung 68 ergibt.

Die beiden gabelförmigen Enden 70 und 74 lassen sich formschlüssig auf das Griffteil 61 bzw. dessen feststehende Branche 62 und den konischen Teil des Drehsterns 66 aufclipsen. Während die Verbindung der Arretiervorrichtung 68 über das gabelförmige Ende 74 mit dem konischen Teil des Drehsterns 66 drehbar sein kann, sind die gabelförmigen Zinken des Endes 70 der Arretiervorrichtung 68 so ausgebildet, dass sie an zwei gegenüberliegenden Oberflächen des feststehenden Griffteils 62 zur Anlage kommen und so über dem in eine der Vertiefungen 78 eingerückten Bügel 72 eine Drehbewegung der Kupplungsvorrichtung 66 (Drehstern) blockieren.

Die beiden gabelförmigen Enden 70, 74 weisen mittig im Bereich zwischen den Zinken eine Ausnehmung 71 bzw. 75 auf, die ein elastisches Auseinanderbiegen der Gabelzinken beim Verbinden der Arretiervorrichtung 68 mit dem Griffteil 61 bzw. der Kupplungsvorrichtung 66 erleichtern.

In der Draufsicht der Figur 2B wird noch deutlicher, wie der mittlere Abschnitt 72 der Arretiervorrichtung 68 von oben in eine Vertiefung 78 des Drehsterns 66 einrückt.

Hier wird auch die Anlage der gabelförmigen Enden des Endabschnitts 70 der Arretiervorrichtung 68 im Klemmsitz an gegenüberliegenden Oberflächen der feststehenden Griffbranche 62 veranschaulicht.

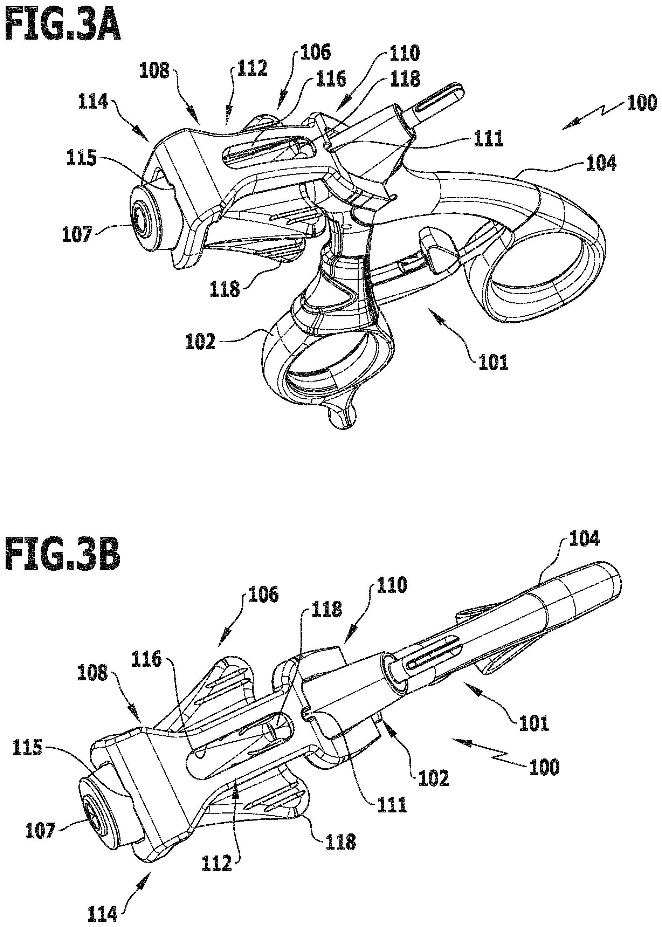

Die Figuren 3A und 3B zeigen in ähnlicher Weise wie die Figuren 2A und 2B das chirurgische Rohrschaftinstrument der vorliegenden Erfindung, wobei der Schaft mit dem Werkzeug wiederum weggelassen ist.

Das erfindungsgemäße Instrument 100 der Figuren 3A und 3B weist ein Griffteil 101 mit einer feststehenden Griffbranche 102 und einer daran verschwenkbar gelagerten Griffbranche 104 auf.

Distal trägt die feststehende Griffbranche 102 eine Kupplungsvorrichtung 106, die die Form eines Drehsterns aufweist. Eine Öffnung 107 am distalen Ende der Kupplungsvorrichtung 106 ermöglicht, dass ein Schaft eingesteckt und drehfest mit der Kupplungsvorrichtung 106 verbunden werden kann. Zusammen mit der Kupplungsvorrichtung 106 ist der Schaft dann wieder gegenüber dem Griffteil 101 drehbar, und diese Drehbarkeit lässt sich wiederum über eine Arretiervorrichtung 108 in vorgegebenen Winkelpositionen blockieren.

Der grundlegende Aufbau der Arretiervorrichtung 108 entspricht in Vielem der Vorrichtung 68 der Figuren 2A und 2B, weshalb im Wesentlichen auf die Beschreibung dort verwiesen werden kann.

So sind die proximalen und distalen Enden 110, 114 der Arretiervorrichtung 108 als gabelförmige Enden ausgebildet, entsprechend den Enden 70 bzw. 74 der Arretiervorrichtung 68 der Figuren 2A und 2B.

Ein elastisches Auseinanderbiegen der Gabelzinken der proximalen und distalen Enden 110, 114 der Arretiervorrichtung 108 beim Montieren am Instrument 100 wird durch die Ausnehmungen 111 bzw. 115 im Bereich zwischen den Gabelzinken erleichtert.

Anders als bei den Ausführungsbeispielen in Figuren 2A und 2B ist bei der Arretiervorrichtung 108 der mittlere Abschnitt 112 mit zwei parallelen Bügeln ausgebildet, die zwischen sich eine Öffnung 116 bilden, in die ein Vorsprung 118 des Drehsterns 106 einrücken kann.

Bei einer im Klemmsitz an der Kupplungsvorrichtung 106 und dem Griffteil 101 platzierten Arretiervorrichtung 108 ist wiederum die Drehposition der Kupplungsvorrichtung 106 (Drehstern) gegenüber dem Griffteil 101 in einer vorgegebenen Winkellage fixiert.

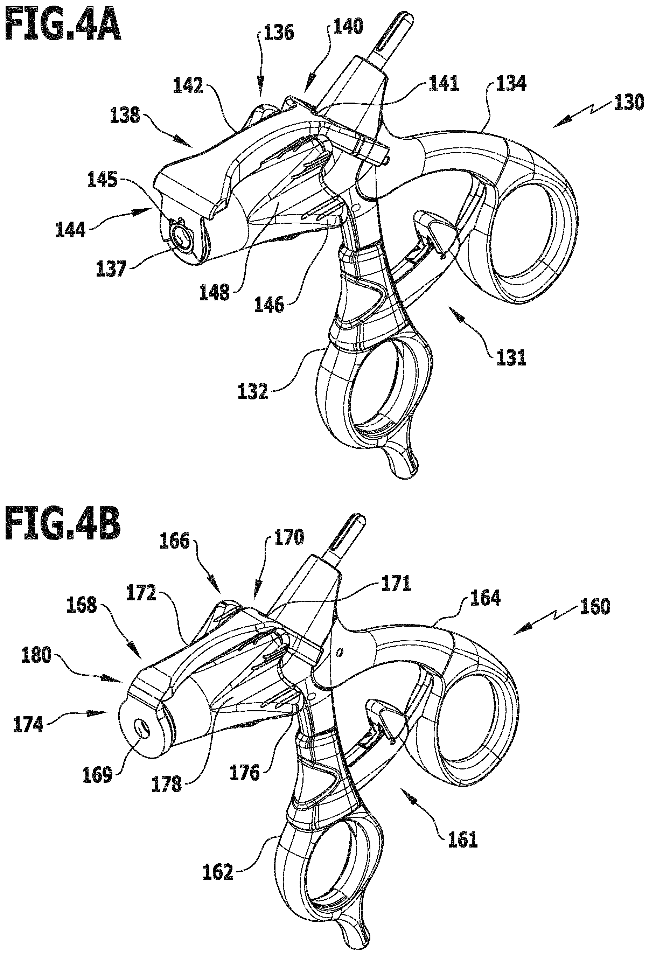

Die Figuren 4A und 4B zeigen in ähnlicher Weise zwei weitere Varianten des chirurgischen Rohrschaftinstruments 130 der vorliegenden Erfindung, wobei der Schaft mit dem Werkzeug wiederum weggelassen ist.

Das Griffteil 131 der Figur 4A weist eine feststehende Griffbranche 132 und eine daran verschwenkbar gelagerte Griffbranche 134 auf.

Distal ist an der feststehenden Griffbranche 132 eine Kupplungsvorrichtung 136, in Form eines Drehsterns drehbar gelagert; eine Öffnung 137 am distalen Ende der Kupplungsvorrichtung 136 ermöglicht, dass ein Schaft eingesteckt und drehfest mit der Kupplungsvorrichtung 136 verbunden werden kann. Zusammen mit der Kupplungsvorrichtung 136 ist der Schaft dann wieder gegenüber dem Griffteil 131 drehbar, und diese Drehbarkeit lässt sich wiederum über eine Arretiervorrichtung 138 in vorgegebenen Winkelpositionen blockieren.

Der grundlegende Aufbau der Arretiervorrichtung 138 entspricht in vielen Einzelheiten der Vorrichtung 68 der Figuren 2A und 2B, weshalb im Wesentlichen auf die Beschreibung dort verwiesen werden kann.

Prinzipiell weist die Arretiervorrichtung 130 ebenfalls gabelförmig ausgebildete proximale und distale Enden 140, 144 und einen dazwischen liegenden Bügel 142 auf.

Ein elastisches Auseinanderbiegen der Gabelzinken der proximalen und distalen Enden 140, 144 beim Montieren der Arretiervorrichtung 138 am Instrument 130 wird durch die Ausnehmungen 141 bzw. 145 erleichtert.

Im montierten Zustand der Arretiervorrichtung 138 greift der Bügel in eine Vertiefung 148 zwischen zwei Vorsprüngen 146 ein.

Anders als bei dem Ausführungsbeispiel der Figuren 2A und 2B ist bei der Arretiervorrichtung 138 das distale Ende 144 so ausgebildet, dass es auf das proximale Ende des Schafts im Bereich der Öffnung 137 aufgeclipst werden kann.

Bei einer im Klemmsitz platzierten Arretiervorrichtung 138 ist wiederum die Drehposition der Kupplungsvorrichtung 136 (Drehstern) gegenüber dem Griffteil 131 in einer vorgegebenen Winkellage fixiert.

Die Figur 4B zeigt ein chirurgisches Rohrschaftinstrument 160 der vorliegenden Erfindung, wobei der Schaft mit dem Werkzeug wiederum weggelassen ist.

Das Griffteil 161 der Figur 4B weist eine feststehende Griffbranche 162 und eine daran verschwenkbar gelagerte Griffbranche 164 auf.

Distal trägt die feststehende Griffbranche 162 eine Kupplungsvorrichtung 166, in Form eines Drehsterns drehbar gelagert. In eine Öffnung 169 am distalen Ende der Kupplungsvorrichtung 166 lässt sich wiederum ein Schaft einstecken und drehfest mit der Kupplungsvorrichtung 166 verbinden. Zusammen mit der Kupplungsvorrichtung 166 ist der Schaft dann wieder gegenüber dem Griffteil 161 drehbar, und diese Drehbarkeit lässt sich wiederum über eine Arretiervorrichtung 168 in vorgegebenen Winkelpositionen blockieren.

Der grundlegende Aufbau der Arretiervorrichtung 168 entspricht in vielen Einzelheiten der Vorrichtung 138 der Figur 4A, weshalb im Wesentlichen auf die Beschreibung dort verwiesen werden kann. So ist das proximale Ende der Arretiervorrichtung 168 als gabelförmiges Ende 170 ausgebildet, von dem aus sich ein Bügel 172 zum distalen Ende 174 der Arretiervorrichtung 168 erstreckt.

Die Kupplungsvorrichtung 166 hat wieder die Form eines Drehsterns mit Vorsprüngen 176 und dazwischen ausgebildeten Vertiefungen 178, in die der Bügel 172 einrücken kann.

Anders als bei dem Ausführungsbeispiel der Figur 4A ist bei der Arretiervorrichtung 168 das distale Ende 174 nicht gabelförmig, sondern als Ringscheibe ausgebildet, deren zentrale Durchgangsöffnung mit der Öffnung 169 fluchtet und so den in die Öffnung 169 der Kupplungsvorrichtung 166 eingesetzten Schaft vollständig umgibt. Damit ist die Arretiervorrichtung 168 im zusammengebauten Zustand des Instruments 160 an diesem unverlierbar gehalten.

Bei einem im Klemmsitz platzierten proximalen Ende 170 der Arretiervorrichtung 168 ist wiederum die Drehposition der Kupplungsvorrichtung 166 (Drehstern) gegenüber dem Griffteil 161 in einer vorgegebenen Winkellage fixiert.

Zum einfacheren Lösen des Klemmsitzes des proximalen Endes 170 der Arretiervorrichtung 168 am Griffteil 161 ist der Bügel 172 über eine gelenkige Verbindung 180 an dem distalen Ende 174 der Arretiervorrichtung 168 gehalten. Der Bügel 172 kann so zusammen mit dem proximalen Ende 170 der Arretiervorrichtung 168 nach oben geschwenkt werden, während das distale Ende 174 am Schaft fixiert bleibt. Nach dem Einstellen der gewünschten Drehposition des Drehsterns 166 zusammen mit dem Schaft gegenüber dem Griffteil 161 wird der Bügel 172 mit dem daran gehaltenen proximalen Ende 170 der Arretiervorrichtung 168 zurück in die in der Figur 4B gezeigte Position verschwenkt. Das proximale Ende 170 kommt im Klemmsitz an der feststehenden Griffbranche 162 zur Anlage, der Bügel 172 rückt in eine Ausnehmung 178 ein und blockiert damit eine weitere Drehbewegung des Drehsterns 166. Die Fixierung des proximalen Endes 170 der Arretiervorrichtung 168 am Griffteil 161 wird wieder durch eine Ausnehmung 171 im Bereich zwischen den Gabelzinken erleichtert.

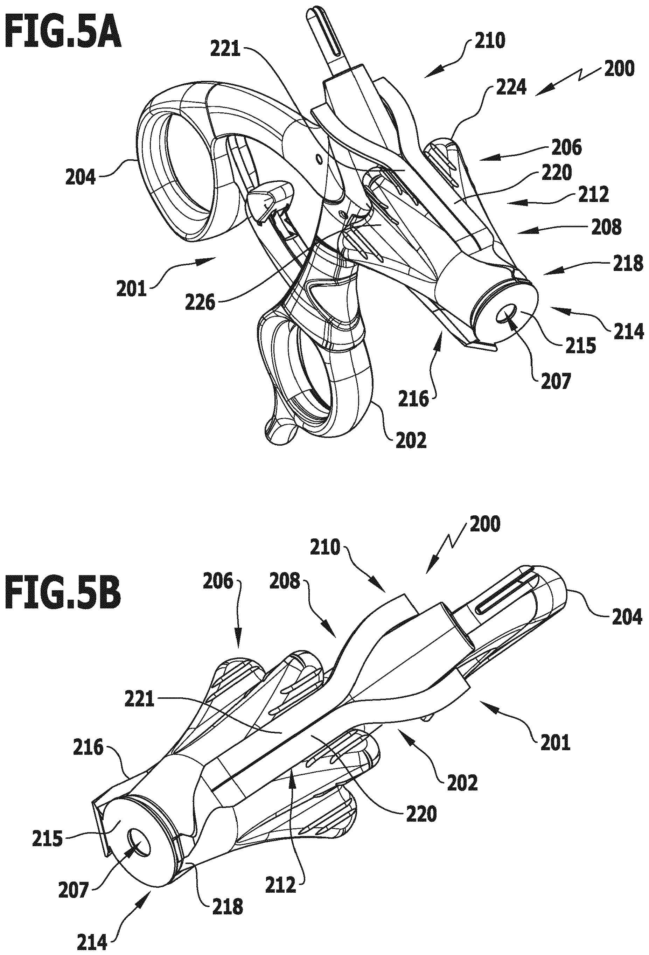

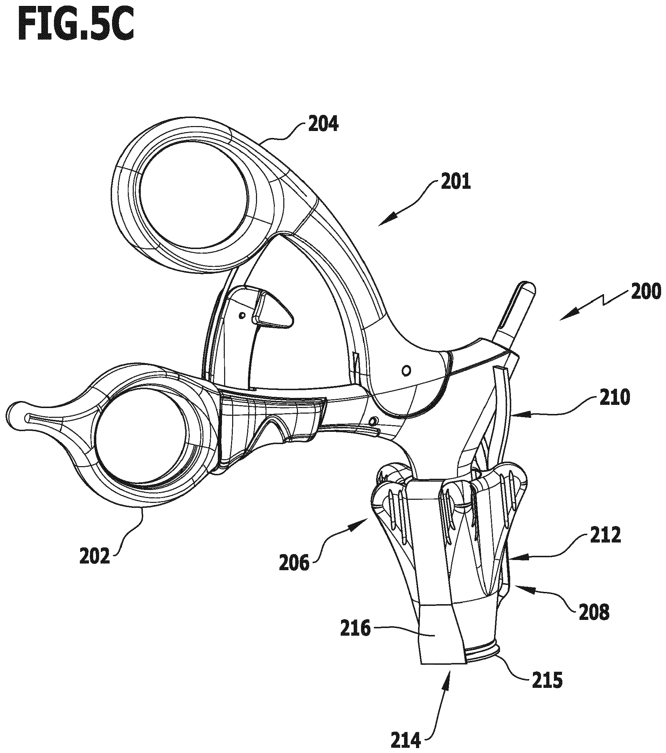

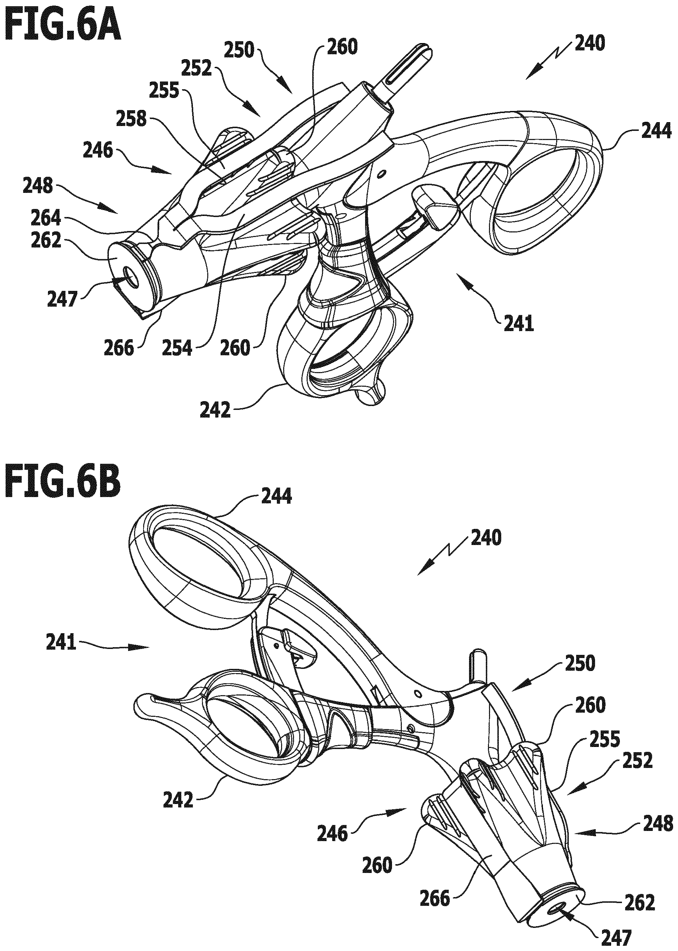

Während die Arretiervorrichtungen der Figuren 2 bis 4 vorzugsweise als Kunststoffspritzgussteile gefertigt werden, werden die nachfolgend im Zusammenhang mit den Figuren 5 und 6 beschriebenen Arretiervorrichtungen vorzugsweise aus einem Flächenmaterial, insbesondere einem Metallblech, ausgestanzt.

Die Figuren 5A bis 5C zeigen ein chirurgisches Rohrschaftinstrument 200 der vorliegenden Erfindung, wobei der Schaft mit dem Werkzeug in der Darstellung wiederum weggelassen ist.

Das Griffteil 201 der Figuren 5A bis 5C weist eine feststehende Griffbranche 202 und eine daran verschwenkbar gelagerte Griffbranche 204 auf.

Distal trägt die feststehende Griffbranche 202 eine Kupplungsvorrichtung 206, in Form eines Drehsterns drehbar gelagert. Eine Öffnung 207 am distalen Ende der Kupplungsvorrichtung 206 nimmt einen Schaft auf, der so drehfest mit der Kupplungsvorrichtung 206 verbunden werden kann. Zusammen mit der Kupplungsvorrichtung 206 ist der Schaft dann wieder gegenüber dem Griffteil 201 drehbar, und diese Drehbarkeit lässt sich wiederum über eine Arretiervorrichtung 208 in vorgegebenen Winkelpositionen blockieren.

Die Arretiervorrichtung 208 ist mit einem gabelförmigen proximalen Ende 210 ausgebildet, an das sich ein Bügel 212 anschließt, der schwenkbar an einer am distalen Ende 214 ausgeformten Ringscheibe 215 gehalten ist. Die zentrale Durchgangsöffnung der Ringscheibe 215 fluchtet mit der Öffnung 207 der Kupplungsvorrichtung 206.

Die Ringscheibe 215 ist an ihrem Umfang gegenüber liegend zu der schwenkbaren Verbindung 218 mit einem Halteelement 216 verbunden, welches sich radial gegenüber liegend zu dem Bügel 212 an der Kupplungsvorrichtung 206 festlegen lässt, so dass die Ringscheibe 215 in ihrer Lage fixiert ist.

Der Bügel 212 wird von zwei parallel aneinander anliegenden Blechstreifen 220, 221 gebildet, die zum proximalen Ende 210 der Arretiervorrichtung 208 hin zu einer gabelförmigen Konfiguration von einander weg gebogen sind.

Die Kupplungsvorrichtung 206 ist auch bei dieser Ausführungsform als Drehstern mit Vorsprüngen 224 und dazwischen liegenden Ausnehmungen 226 ausgebildet und der Bügel 212 greift im montierten Zustand der Arretiervorrichtung 208 in eine der Ausnehmungen 226 ein. Sobald der Bügel 212 in eine der Ausnehmungen 226 einrückt, werden die Blechstreifen 220, 221 aneinander anliegend fixiert und gleichzeitig deren freie Enden, die das proximale Ende 210 der Arretiervorrichtung 208 bilden, zur Anlage im Klemmsitz an gegenüberliegenden Oberflächen des Griffteils 201 gezwungen.

Vorzugsweise ist die Arretiervorrichtung 208 einstückig aus einem Flächenmaterial ausgestanzt.

Die Figuren 6A und 6B zeigen ein erfindungsgemäßes chirurgisches Rohrschaftinstrument 240 mit einem Griffteil 241, wobei der Schaft mit dem Werkzeug in der Darstellung der Einfachheit halber weggelassen wurde.

Das Griffteil 241 weist eine feststehende Griffbranche 242 und eine daran schwenkbar gelagerte Griffbranche 244 auf. Distal trägt das Griffteil 241 eine drehbar gelagerte Kupplungsvorrichtung 246 mit einer Öffnung 247, in die ein Schaft zur drehfesten Verbindung eingerückt werden kann.

Die Drehposition der Kupplungsvorrichtung 246 gegenüber dem Griffteil 241 lässt sich mittels einer Arretiervorrichtung 248 festlegen.

Der grundlegende Aufbau der Arretiervorrichtung 248 entspricht überwiegend der Vorrichtung 208 der Figuren 5A bis 5C, weshalb auch auf die Beschreibung dort verwiesen werden kann. So ist das proximale Ende 250 der Arretiervorrichtung 248 gabelförmig ausgebildet und umgreift im Klemmsitz die feststehende Griffbranche 242.

Anders als bei dem Ausführungsbeispiel der Figuren 5A bis 5C ist bei der Arretiervorrichtung 248 der sich an das proximale Ende 250 anschließende mittlere Abschnitt 252 aus zwei parallelen Bügeln 254, 255 gebildet, die zwischen sich eine spaltförmige Öffnung 258 bilden, in die ein Vorsprung 260 der als Drehstern ausgebildeten Kupplungsvorrichtung 246 einrücken kann.

Bei einer im Klemmsitz platzierten Arretiervorrichtung 248 ist wiederum die Drehposition der Kupplungsvorrichtung 246 (Drehstern) gegenüber dem Griffteil 241 in einer vorgegebenen Winkellage fixiert.

An ihrem distalen Ende ist die Arretiervorrichtung 248 mit einer Ringscheibe 262 versehen, deren zentrale Durchgangsöffnung mit der Öffnung 247 fluchtet. Die Ringscheibe 262 ist mit dem mittleren Abschnitt 252 über eine Schwenkverbindung 264 verbunden.

Radial gegenüber liegend zu der Schwenkverbindung 264 ist an der Ringscheibe 262 ein Halteelement 266 angeformt, mit dem die Ringscheibe 262 als distales Ende der Arretiervorrichtung 248 an der Kupplungsvorrichtung 246 festlegbar ist.

## Patentansprüche

1. Chirurgisches Rohrschaftinstrument (60) mit einem Griffteil (61), einem rohrförmigen Schaft (16) mit einem proximalen und einem distalen Ende, mit einem am distalen Ende des Schafts angeordneten Werkzeug (18) sowie mit einem Kraftübertragungselement,
wobei das Kraftübertragungselement in dem Schaft (16) geführt angeordnet ist und zum Übertragen einer Betätigungskraft vom Griffteil (61) auf das Werkzeug (18) zum einen mit dem Griffteil (61) und zum anderen mit dem Werkzeug (18) gekoppelt ist,
wobei der Schaft (16) um seine Längsachse drehbar an dem Griffteil (61) gehalten ist,
wobei das Instrument (60) ferner eine Arretiervorrichtung (68) zum Festlegen des Schafts (16) in einer vorgegebenen Drehposition gegenüber dem Griffteil (61) umfasst, **dadurch gekennzeichnet, dass** das Instrument (60) eine Kupplungsvorrichtung (66) umfasst, die drehbar am Griffteil (61) gehalten ist, wobei das proximale Ende des Schafts (16) lösbar und drehfest an der Kupplungsvorrichtung (66) gehalten ist, wobei die Drehposition der Kupplungsvorrichtung (66) mittels der Arretiervorrichtung (68) gegenüber dem Griffteil (61) entsprechend der vorgegebenen Drehposition des Schafts (16) gegenüber dem Griffteil (61) festlegbar ist,
wobei die Arretiervorrichtung (68) ein proximales und ein distales Ende (70; 74) und einen dazwischen angeordneten Mittelabschnitt (72) aufweist, wobei das distale Ende (74) am proximalen Ende (70) des Schafts (16) oder an der Kupplungsvorrichtung (66) befestigbar ist, wobei das proximale Ende (70) der Arretiervorrichtung (68) an dem Griffteil (61) drehfest befestigbar ist, wobei die Kupplungsvorrichtung (66) und der Mittelabschnitt (72) der Arretiervorrichtung (68) miteinander in Eingriff bringbar sind und wobei zumindest das proximale (70) oder das distale Ende (74) lösbar am Instrument (60) befestigbar ist.

2. Rohrschaftinstrument nach Anspruch 1, **dadurch gekennzeichnet, dass** die Drehposition der Kupplungsvorrichtung (66) mittels der Arretiervorrichtung (68) gegenüber dem Griffteil (61) entsprechend der vorgegebenen Drehposition des Schafts (16) gegenüber dem Griffteil (61) in vorgegebenen Winkelinkrementen festlegbar ist.

3. Rohrschaftinstrument nach Anspruch 1 oder 2, **dadurch gekennzeichnet, dass** die Arretiervorrichtung (68) lösbar am Instrument (60) befestigbar ist.

4. Rohrschaftinstrument nach einem der Ansprüche 1 bis 3, **dadurch gekennzeichnet, dass** das distale Ende (74) der Arretiervorrichtung (68) mit dem Schaft (16) oder der Kupplungsvorrichtung (66) drehbar verbunden ist.

5. Rohrschaftinstrument nach einem der Ansprüche 1 bis 4, **dadurch gekennzeichnet, dass** eines der Enden der Arretiervorrichtung (68) schwenkbar mit dem Mittelabschnitt (72) verbunden ist.

6. Rohrschaftinstrument nach einem der Ansprüche 1 bis 5, **dadurch gekennzeichnet, dass** die Arretiervorrichtung (68) mit einem ihrer Enden fest mit dem Instrument (60) verbunden ist.

7. Rohrschaftinstrument nach einem der Ansprüche 1 bis 6, **dadurch gekennzeichnet, dass** das proximale Ende (70) der Arretiervorrichtung (68) im Klemmsitz am Griffteil (61) gehalten ist und/oder dass das distale Ende (74) der Arretiervorrichtung (68) im Klemmsitz am Schaft (16) oder an der Kupplungsvorrichtung (66) gehalten ist.

8. Rohrschaftinstrument nach einem der Ansprüche 1 bis 7, **dadurch gekennzeichnet, dass** das proximale Ende (70) der Arretiervorrichtung (68) gabelförmig ausgebildet ist und an zwei gegenüberliegenden Oberflächen des Griffteils (61) in Anlage bringbar ist.

9. Rohrschaftinstrument nach einem der Ansprüche 1 bis 8, **dadurch gekennzeichnet, dass** das distale Ende (74) der Arretiervorrichtung (68) gabelförmig ausgebildet ist und die Kupplungsvorrichtung (66) oder den Schaft (16) in einem Umfangswinkel von mehr als 180° umgreift.

10. Rohrschaftinstrument nach einem der Ansprüche 1 bis 9, **dadurch gekennzeichnet, dass** der Mittelabschnitt (72) der Arretiervorrichtung (68) ein Bügelteil aufweist, welches in eine im Wesentlichen komplementär ausgebildete Ausnehmung der Kupplungsvorrichtung (66) einrückbar ist.

11. Rohrschaftinstrument nach einem der Ansprüche 1 bis 9, **dadurch gekennzeichnet, dass** der Mittelabschnitt (72) der Arretiervorrichtung (68) eine Ausnehmung, optional eine durchgehende Ausnehmung, aufweist, in welche ein Vorsprung der Kupplungsvorrichtung (66) einrückbar ist, wobei die Kupplungsvorrichtung (66) optional mehrere, insbesondere vier bis sieben, sternförmig angeordnete Vorsprünge aufweist.

12. Rohrschaftinstrument nach einem der Ansprüche 1 bis 11, **dadurch gekennzeichnet, dass** die Arretiervorrichtung (68) als ein Kunststoffteil, insbesondere als Spritzgussteil, oder dass die Arretiervorrichtung (68) als Blechteil ausgebildet ist.

13. Rohrschaftinstrument nach einem der Ansprüche 1 bis 12, **dadurch gekennzeichnet, dass** der Schaft (16) einen im Wesentlichen geraden mittleren Abschnitt und einen gegenüber dem mittleren Abschnitt abgewinkelten distalen Abschnitt aufweist, wobei optional der distale Abschnitt im Wesentlichen kreisbogenförmig ausgebildet ist.

14. Rohrschaftinstrument nach Anspruch 13, **dadurch gekennzeichnet, dass** der Schaft (16) einen gegenüber dem mittleren Abschnitt abgewinkelten proximalen Abschnitt aufweist, wobei optional der proximale Abschnitt eine im Wesentlichen s-förmige Gestalt aufweist, wobei vorzugsweise der proximale Endbereich des proximalen Abschnitts des Schafts (16) in einem spitzen Winkel zur Längsachse des mittleren Abschnitts des Schafts ausgerichtet ist.

15. Rohrschaftinstrument nach Anspruch 14, **dadurch gekennzeichnet, dass** das Längenverhältnis des proximalen zum mittleren Abschnitt des Schafts (16) ca. 1:2 bis ca. 1:3 beträgt und/oder dass das Längenverhältnis des mittleren zum distalen Abschnitt ca. 2:1 bis ca. 3:1 beträgt.

## Claims

1. Surgical tubular shaft instrument (60), comprising a handle part (61), a tubular shaft (16) having a proximal and a distal end, a tool (18) arranged at the distal end of the shaft and a force transmission element,
wherein the force transmission element is arranged in the shaft (16) in guided relation thereto and is coupled to the handle part (61) on the one hand and to the tool (18) on the other hand for transmitting an actuating force from the handle part (61) to the tool (18),
wherein the shaft (16) is held on the handle part (61) for rotation about its longitudinal axis,
wherein the instrument (60) further comprises a locking device (68) for fixing the shaft (16) in a predetermined rotational position relative to the handle part (61),
**characterized in that** the instrument (60) comprises a coupling device (66) rotatably held on the handle part (61), wherein the proximal end of the shaft (16) is releasably and rotationally fixedly held on the coupling device (66), wherein the rotational position of the coupling device (66) is fixable relative to the handle part (61) by way of the locking device (68) in a manner corresponding to the predetermined rotational position of the shaft (16) relative to the handle part (61),
wherein the locking device (68) has a proximal and a distal end (70; 74) and a mid-section (72) arranged therebetween, wherein the distal end (74) is fixable to the proximal end (70) of the shaft (16) or to the coupling device (66), wherein the proximal end (70) of the locking device (68) is fixable to the handle part (61) in rotationally fixed relation thereto, wherein the coupling device (66) and the mid-section (72) of the locking device (68) are engageable with each other and wherein at least the proximal (70) or the distal end (74) is releasably fixable to the instrument (60).

2. Tubular shaft instrument in accordance with claim 1, **characterized in that** the rotational position of the coupling device (66) is fixable relative to the handle part (61) by way of the locking device (68) in a manner corresponding to the predetermined rotational position of the shaft (16) relative to the handle part (61) in predetermined angular increments.

3. Tubular shaft instrument in accordance with claim 1 or 2, **characterized in that** the locking device (68) is releasably fixable to the instrument (60).

4. Tubular shaft instrument in accordance with any one of claims 1 to 3, **characterized in that** the distal end (74) of the locking device (68) is rotatably connected to the shaft (16) or the coupling device (66).

5. Tubular shaft instrument in accordance with any one of claims 1 to 4, **characterized in that** one of the ends of the locking device (68) is pivotally connected to the mid-section (72).

6. Tubular shaft instrument in accordance with any one of claims 1 to 5, **characterized in that** the locking device (68) is fixedly connected at one of its ends to the instrument (60).

7. Tubular shaft instrument in accordance with any one of claims 1 to 6, **characterized in that** the proximal end (70) of the locking device (68) is held in a clamping fit on the handle part (61) and/or **in that** the distal end (74) of the locking device (68) is held in a clamping fit on the shaft (16) or on the coupling device (66).

8. Tubular shaft instrument in accordance with any one of claims 1 to 7, **characterized in that** the proximal end (70) of the locking device (68) is of forked configuration and capable of being brought into contact against two opposite surfaces of the handle part (61).

9. Tubular shaft instrument in accordance with any one of claims 1 to 8, **characterized in that** the distal end (74) of the locking device (68) is of forked configuration and engages around the coupling device (66) or the shaft (16) at a circumferential angle of more than 180°.

10. Tubular shaft instrument in accordance with any one of claims 1 to 9, **characterized in that** the mid-section (72) of the locking device (68) comprises a bow-shaped part which is engageable in a recess of the coupling device (66) that is configured substantially complementarily thereto.

11. Tubular shaft instrument in accordance with any one of claims 1 to 9, **characterized in that** the mid-section (72) of the locking device (68) comprises a recess, optionally a continuous recess, in which a projection of the coupling device (66) is engageable, optionally wherein the coupling device (66) has a plurality, in particular four to seven, projections in a star-shaped arrangement.

12. Tubular shaft instrument in accordance with any one of claims 1 to 11, **characterized in that** the locking device (68) is formed as a plastic part, in particular as an injection-moulded part, or **in that** the locking device (68) is formed as a sheet metal part.

13. Tubular shaft instrument in accordance with any one of claims 1 to 12, **characterized in that** the shaft (16) has a substantially straight middle section and a distal section in an angled relationship to the middle section, optionally wherein the distal section is of substantially circular arc shaped configuration.

14. Tubular shaft instrument in accordance with claim 13, **characterized in that** the shaft (16) has a proximal section in an angled relationship to the middle section, optionally wherein the proximal section has a substantially s-like shape, preferably wherein the proximal end portion of the proximal section of the shaft (16) is oriented at an acute angle to the longitudinal axis of the middle section of the shaft.

15. Tubular shaft instrument in accordance with claim 14, **characterized in that** the length ratio of the proximal section to the middle section of the shaft (16) is approximately 1:2 to approximately 1:3 and/or **in that** the length ratio of the middle section to the distal section is approximately 2:1 to approximately 3:1.

## Revendications

1. Instrument à tige tubulaire chirurgical (60) avec une poignée (61), une tige en forme de tube (16) avec une extrémité proximale et une extrémité distale, avec un outil (18) disposé à l'extrémité distale de la tige ainsi qu'avec un élément de transmission de force,
où l'élément de transmission de force est disposé de manière guidée dans la tige (16) et est couplé d'une part avec la poignée (61) et d'autre part avec l'outil (18) pour la transmission d'une force d'actionnement de la poignée (61) à l'outil (18),
où la tige (16) est maintenue sur la poignée (61) de manière à pouvoir tourner autour de son axe longitudinal,
où l'instrument (60) comprend en outre un dispositif d'arrêt (68) pour la fixation de la tige (16) dans une position de rotation prédéterminée par rapport à la poignée (61),
**caractérisé en ce que**
l'instrument (60) comprend un dispositif de couplage (66) qui est maintenu sur la poignée (61) de manière à pouvoir tourner, où l'extrémité proximale de la tige (16) est maintenue sur le dispositif de couplage (66) de manière amovible et fixe en rotation, où la position de rotation du dispositif de couplage (66) peut être établie au moyen du dispositif d'arrêt (68) par rapport à la poignée (61) d'une manière correspondant à la position de rotation prédéterminée de la tige (16) par rapport à la poignée (61),
où le dispositif d'arrêt (68) présente une extrémité proximale et une extrémité distale (70 ; 74) et une section centrale (72) disposée entre elles, où l'extrémité distale (74) peut être fixée à l'extrémité proximale (70) de la tige (16) ou au dispositif de couplage (66), où l'extrémité proximale (70) du dispositif d'arrêt (68) peut être fixée de manière fixe en rotation à la poignée (61), où le dispositif de couplage (66) et la section centrale (72) du dispositif d'arrêt (68) peuvent être amenés en prise l'un avec l'autre et où au moins l'extrémité proximale (70) ou l'extrémité distale (74) peut être fixée de manière amovible à l'instrument (60).

2. Instrument à tige tubulaire selon la revendication 1, **caractérisé en ce que** la position de rotation du dispositif de couplage (66) peut être établie au moyen du dispositif d'arrêt (68) par rapport à la poignée (61) d'une manière correspondant à la position de rotation prédéterminée de la tige (16) par rapport à la poignée (61) par incréments angulaires prédéterminés.

3. Instrument à tige tubulaire selon la revendication 1 ou 2, **caractérisé en ce que** le dispositif d'arrêt (68) peut être fixé à l'instrument (60) de manière amovible.

4. Instrument à tige tubulaire selon l'une des revendications 1 à 3, **caractérisé en ce que** l'extrémité distale (74) du dispositif d'arrêt (68) est reliée avec la tige (16) ou avec le dispositif de couplage (66) de manière à pouvoir tourner.

5. Instrument à tige tubulaire selon l'une des revendications 1 à 4, **caractérisé en ce que** l'une des extrémités du dispositif d'arrêt (68) est reliée avec la section centrale (72) de manière à pouvoir pivoter.

6. Instrument à tige tubulaire selon l'une des revendications 1 à 5, **caractérisé en ce que** le dispositif d'arrêt (68) est relié par l'une de ses extrémités avec l'instrument (60) de manière fixe.

7. Instrument à tige tubulaire selon l'une des revendications 1 à 6, **caractérisé en ce que** l'extrémité proximale (70) du dispositif d'arrêt (68) est maintenue en position de serrage sur la poignée (61) et/ou **en ce que** l'extrémité distale (74) du dispositif d'arrêt (68) est maintenue en position de serrage sur la tige (16) ou sur le dispositif de couplage (66).

8. Instrument à tige tubulaire selon l'une des revendications 1 à 7, **caractérisé en ce que** l'extrémité proximale (70) du dispositif d'arrêt (68) est formée en forme de fourche et peut être amenée en appui sur deux surfaces opposées de la poignée (61).

9. Instrument à tige tubulaire selon l'une des revendications 1 à 8, **caractérisé en ce que** l'extrémité distale (74) du dispositif d'arrêt (68) est formée en forme de fourche et enveloppe le dispositif de couplage (66) ou la tige (16) sous un angle périphérique de plus de 180°.

10. Instrument à tige tubulaire selon l'une des revendications 1 à 9, **caractérisé en ce que** la section centrale (72) du dispositif d'arrêt (68) présente une partie en étrier qui peut être rentrée dans un évidement du dispositif de couplage (66) formé de manière sensiblement complémentaire.

11. Instrument à tige tubulaire selon l'une des revendications 1 à 9, **caractérisé en ce que** la section centrale (72) du dispositif d'arrêt (68) présente un évidement, éventuellement un évidement traversant, dans lequel une protubérance du dispositif de couplage (66) peut être rentrée, où le dispositif de couplage (66) présente éventuellement plusieurs, en particulier quatre à sept, protubérances disposées en forme d'étoile.

12. Instrument à tige tubulaire selon l'une des revendications 1 à 11, **caractérisé en ce que** le dispositif d'arrêt (68) est formé sous forme d'une pièce en matière synthétique, en particulier sous forme d'une pièce moulée par injection, ou **en ce que** le dispositif d'arrêt (68) est formé sous forme d'une pièce en tôle.

13. Instrument à tige tubulaire selon l'une des revendications 1 à 12, **caractérisé en ce que** la tige (16) présente une section moyenne sensiblement rectiligne et une section distale coudée par rapport à la section moyenne, où éventuellement la section distale est formée sensiblement en forme d'arc de cercle.

14. Instrument à tige tubulaire selon la revendication 13, **caractérisé en ce que** la tige (16) présente une section proximale coudée par rapport à la section moyenne, où éventuellement la section proximale présente une configuration sensiblement en forme de s, où de préférence le domaine d'extrémité proximal de la section proximale de la tige (16) est orienté sous un angle aigu par rapport à l'axe longitudinal de la section moyenne de la tige.

15. Instrument à tige tubulaire selon la revendication 14, **caractérisé en ce que** le rapport des longueurs de la section proximale à la section moyenne de la tige (16) est d'environ 1:2 à environ 1:3 et/ou **en ce que** le rapport des longueurs de la section moyenne à la section distale est d'environ 2:1 à environ 3:1.
